# EUROPÄISCHE PATENTSCHRIFT

(11) **EP 2 953 575 B1**
(45) Veröffentlichungstag und Bekanntmachung des Hinweises auf die Patenterteilung: **28.11.2018**
(21) Anmeldenummer: 14705724.4
(22) Anmeldetag: 05.02.2014
(51) Int. Cl.: A61C 8/00

(54) **KERAMIKKÖRPER, INSBESONDERE ZUR VERWENDUNG ALS DENTALIMPLANTAT**
CERAMIC BODY, IN PARTICULAR FOR USE AS A DENTAL IMPLANT
CORPS CÉRAMIQUE CONÇU EN PARTICULIER POUR ÊTRE UTILISÉ EN TANT Q'IMPLANT DENTAIRE

(30) Priorität: 05.02.2013 DE 102013201885
(43) Veröffentlichungstag der Anmeldung: 16.12.2015
(73) Patentinhaber: Brodbeck, Urs, 8703 Erlenbach (CH); Schlee, Markus, 91301 Forchheim (DE); Zipprich, Holger, 64342 Malchen (DE)
(72) Erfinder: BRODBECK, Urs, 8703 Erlenbach (CH)
(74) Vertreter: Tergau & Walkenhorst Patentanwälte PartGmbB
(86) Internationale Anmeldenummer: PCT/EP2014/052272
(87) Internationale Veröffentlichungsnummer: WO 2014/122189

(56) Entgegenhaltungen:
- WO-A1-03/045268
- WO-A1-2008/011948

## Beschreibung

Die Erfindung betrifft einen Keramikkörper, insbesondere zur Verwendung in einem Knochenimplantat, insbesondere als Dentalimplantat. Sie betrifft weiter ein Knochenimplantat, insbesondere ein Dentalimplantat, mit einem derartigen Keramikkörper.

In diesem Zusammenhang wird die WO 03/045268 A1 genannt. Knochenimplantate und insbesondere Dentalimplantate sind in vielfältigen Formen bekannt. Dentalimplantate werden meist durch Einschrauben an Stelle eines extrahierten oder ausgefallenen Zahnes in den Kieferknochen eingesetzt, um dort nach einer Einheilphase von drei bis vier Monaten ein als Zahnersatz dienendes prothetisches Aufbauteil oder eine Krone zu halten. Dazu ist ein derartiges Zahnimplantat, wie auch ein Knochenimplantat im Allgemeinen, üblicherweise als geeignet geformter Metall- oder Keramikkörper ausgebildet und in der Art eines Stiftes geformt. Dieser weist am apikalen Ende ein zumeist selbstschneidendes Schraubengewinde auf, mit welchem der Stift in das entsprechend präparierte Implantatbett eingesetzt wird.
In der Regel werden Dentalimplantate aus Titan, Zirkon, Niob oder Tantal oder aus gewebeverträglichen Legierungen, die eines dieser Elemente als Hauptbestandteil enthalten, hergestellt. Darüber hinaus werden Dentalimplantate auch aus Keramiken hergestellt. Die verwendeten Keramiken sind meistens Keramiken auf Zirkonoxidbasis, bei welchen vorzugsweise mittels der Beimischung von Yttriumoxid die tetragonale Phase stabilisiert ist (TZP, TZP-A mit Aluminiumoxidanteilen), oder die durch die, meist zusätzliche, Beimischung von Aluminiumoxid, Aluminiumoxid verstärkt sind (ATZ-Keramiken). Es sind aber auch Dentalimplantate auf Aluminiumoxidbasis bekannt.

Aus der nicht vorveröffentlichten deutschen Patentanmeldung mit dem Aktenzeichen 10 2012 022 593.8, ist ein Behandlungselement, insbesondere zur Verwendung mit einem Implantat-Teil, sowie ein Verfahren zum Reinigen eines Dental-Implantat-Teils bekannt. Eine derartige Reinigung eines Implantat-Teils kann wünschenswert oder erforderlich sein, um den Erhalt des inserierten Implantats in der Knochensubstanz zu gewährleisten. An der von Gewebe und Gewebeflüssigkeit umschlossenen festen Oberfläche von Implantaten kann sich nämlich ein Biofilm bilden, der von Bakterien besiedelt wird, die letztlich zu chronischen und wiederkehrenden Infektionen führen können. Dieses Erkrankungsbild wird bei Dentalimplantaten als Periimplantitis bezeichnet. Insbesondere im dentalen Bereich ist ähnlich wie bei der Parodontitis eine Kombination aus vernachlässigter Mundhygiene, Anhaftung von Biofilm an der üblicherweise mikrorauen Oberfläche des Dentalimplantats und weiterer Faktoren ursächlich für das Vollbild der Periimplantitis, welche sich durch eine zunehmende Belastung und Zerstörung des Hart- und Weichgewebes auszeichnet. Die Bereiche, an denen sich das Hart- und/oder Weichgewebe zurückzieht, werden dabei in der Regel mit einem Biofilm überzogen.
Das in der genannten Anmeldung beschriebene Reinigungsverfahren beruht auf dem Konzept, den die Verunreinigung bildenden Biofilm bzw. die Keime ausgehend von der Implantatoberfläche abzutöten und zu entfernen, ohne dabei die Implantatoberfläche zu beschädigen. Dazu ist ein elektrolytischer Prozess vorgesehen, bei dem Ionen (Kationen und/oder Anionen) mittels elektrostatischer Kräfte durch den Biofilm hindurch befördert werden. Diese Ionen reagieren an der Implantatoberfläche chemisch oder elektrochemisch. Durch diese Reaktionen werden neue Stoffverbindungen geschaffen und/ oder die Ionen selbst und/oder Teile dieser Ionen in den atomaren Zustand überführt. Darüber hinaus besteht zudem die Möglichkeit, dass die Ionen mit dem Oberflächenmaterial reagieren (z. B. Bildung einer Oxidschicht oder Materialabtrag).
Die keimtötende Wirkung dieses Prozesses basiert auf unterschiedlichen Effekten. Zum einen werden durch das Anlegen einer elektrischen Spannung Ionen aus dem Biofilm selbst (auch aus den Bakterien) zur Anode oder Kathode befördert. Dies kann zur Abtötung von Bakterien und Viren führen. Darüber hinaus können die Ionen, während sie den Biofilm passieren, biochemische Reaktionen eingehen, was ebenfalls zur Abtötung von Bakterien und/oder Viren führen kann. Eine weitere Möglichkeit der Abtötung besteht darin, dass die an der Implantatoberfläche neu gebildeten Stoffverbindungen antibakterielle und/oder antivirale und/oder antifungizide Wirkung besitzen. Dies kann natürlich auch passieren, wenn die Ionen in den atomaren Zustand übergehen.

Das in der genannten Anmeldung beschriebene Behandlungselement ist spezifisch dafür ausgelegt, dieses Reinigungsverfahren direkt am inserierten Dentalimplantat durchzuführen, also bevorzugt während sich das Pfostenteil im Knochen im Patientenmund befindet. Dazu ist das Behandlungselement dazu vorgesehen, direkt mit dem inserierten Pfostenteil verbunden zu werden und sodann eine geeignete Behandlungsflüssigkeit, die bei Beaufschlagung mit elektrischem Strom als Basis für den angestrebten elektrolytischen Prozess dienen kann, in unmittelbarer Nähe zum inserierten Pfostenteil in den betroffenen Raumbereich der benachbarten Knochensubstanz auszubringen und mit dem elektrischen Strom zu beaufschlagen. Zum Einsatz dieses Behandlungselements ist jedoch die Herstellung eines sowohl mechanischen als auch elektrischen Kontakts mit dem inserierten Pfostenteil erforderlich.

Der vorliegenden Erfindung liegt die Aufgabe zu Grunde, einen zur Verwendung in einem Knochenimplantat, insbesondere in einem Dentalimplantat, geeigneten Keramikkörper anzugeben, der für die Anwendung des in der genannten Anmeldung beschriebenen Reinigungs- und Behandlungskonzepts geeignet ertüchtig ist. Weiterhin soll ein für die Verwendung des in der genannten Anmeldung beschriebenen Reinigungs- und Behandlungskonzepts besonders geeignetes Knochenimplantat, insbesondere ein Dentalimplantat, mit einem derartigen Keramikkörper angegeben werden.

Bezüglich des Keramikkörpers wird diese Aufgabe erfindungsgemäß gelöst, indem dieser in einem für einen Kontakt mit menschlichem Gewebe vorgesehenen Oberflächenbereich einen spezifischen elektrischen Widerstand von höchstens 10⁻² Ω cm aufweist.

Vorteilhafte Ausgestaltungen der Erfindung sind Gegenstand der Unteransprüche. Weitere und/oder alternative vorteilhafte Ausgestaltungen der Erfindung ergeben sich auch aus der Figurenbeschreibung.

Die Erfindung geht von der Überlegung aus, dass eine elektrolytische Behandlung und Reinigung des Keramikkörpers von Biofilm ermöglicht ist, indem er gezielt für die dazu erforderliche Beaufschlagung mit Strom ertüchtigt wird. Dazu sollte er gerade in den Oberflächenbereichen, in denen die elektrolytische Zersetzung der Behandlungsflüssigkeit erfolgen soll, geeignet leitfähig ausgestaltet werden.
Um dies zu erreichen, kommen grundsätzlich verschiedene Ansätze in Frage. Zum einen kann der Keramikkörper vollständig aus einer elektrisch ausreichend leitfähigen oder einer elektrisch halbleitenden Keramik gefertigt sein.
Alternativ kann der Keramikkörper im für den Kontakt mit menschlichem Gewebe vorgesehenen Oberflächenbereich mit einer elektrisch leitfähigen Beschichtung versehen sein. Beispielsweise ist eine derartige Beschichtung von einem Metall, erfindungsgemäß jedoch von einem elektrisch leitfähigen Kunststoff und/oder von einem elektrisch leitfähigen Kohlenstoff gebildet, oder besonders bevorzugt von einer dotierten Keramik, vorzugsweise von Indiumoxid und/oder Indium-Zinn-Oxid, von Zinkoxid, von Siliziumnitrit, von MoSi₂, von Si₃N₄-MoSi₂-SiC, von ZrO₂/CaO, von Al₂O₃/TiN, von BaTiO₃, von einem Silizid, von einem Nitrid, von einer titanhaltigen Keramik und/oder von einem Titanat. Als metallische Beschichtung kommen z. B. Platin, Gold, Titan, etc. in Frage. Eine metallische Beschichtung könnte allerdings bei der Verwendung in einem Dental-implantat aus ästhetischer, biologischer oder allergologischen Gründen nur bedingt geeignet sein. Im Gegensatz dazu könnten die besonders bevorzugten elektrisch leitfähigen bzw. elektrisch halbleitenden Keramiken gerade bei einem Einsatz in einem Dentalimplantat bei entsprechender Farbgebung in der Beschichtung auch den ästhetischen, biologischen und allergologischen Ansprüchen genügen.
Ein vollkeramisches, an sich elektrisch nicht leitfähiges Dentalimplantat (Aufbauteil/ Pfostenteil) kann mit solch einer elektrischen leitfähigen bzw. elektrisch halbleitenden Keramik beschichtet werden. Diese Beschichtung kann vollständig/gänzlich oder nur in Teilbereichen erfolgen. Die elektrische Kontaktierung erfolgt dabei bevorzugt über die Implantatschulter. Hierfür wäre es günstig, die Beschichtungsdicke an der Kontaktierungsstelle im Bereich der Implantatschulter dicker zu gestalten, z. B. doppelt, fünffach, zehnfach so dick. Würde man das Äußere und das Innere eines Implantats solchermaßen beschichten, wäre auch eine elektrische Kontaktierung über das Innere des Implantats möglich.

Bei der anodischen Beschaltung von Titanimplantaten, insbesondere von Titan Grade IV oder reinerem Titan, ergibt sich eine Titanoxidschichtbildung, welche direkt nach ihrer Entstehung ohne Erhöhung der elektrischen Spannung die elektrochemischen Prozesse an der Oberfläche sehr stark reduziert bzw. unterbindet. Eine anodische Bestromung zur Reinigung ist somit nur sehr kurzfristig bzw./oder einmalig möglich, ohne den Patienten durch die elektrische Spannung zu gefährden. Würde man ein solches Titanimplantat mit einer elektrisch leitfähigen bzw. elektrisch halbleitenden Keramik beschichten, könnte man das Titanoxidwachstum bei anodischer Bestromung verhindern. Auf diese Weise könnte man es ermöglichen, die anodische Bestromung zur Reinigung der Implantatoberfläche lang dauernd zu nutzen. Als weiterer Vorteil einer solchen Oberfläche würde es sich ergeben, dass keine Metallionen aus dem metallischen Implantat herausgelöst werden könnten.

Weiterhin ist auch denkbar, dass einer nicht elektrisch leitfähigen Keramik, welche sich als Implantatmodul eignet, ein elektrisch leitender oder halbleitender Stoff, vorzugsweise auf keramischer und/oder Kohlenstoffbasis beigemischt wird, um eine elektrische Leitfähigkeit oder elektrische Halbleitfähigkeit hervorzurufen.

Besonders bevorzugt wird der Keramikkörper für ein Knochenimplantat, insbesondere als Teil eines Dentalimplantats, verwendet. Dies kann dann entweder von okklusal oder über das Innere elektrisch kontaktiert werden zum Zwecke der Reinigung. Eine elektrische Kontaktierung über eine Reinigungskanüle von außen kann selbstverständlich auch angewendet werden.

Bezüglich des Knochenimplantats wird die genannte Aufgabe gelöst, indem dieses als Keramikkörper der genannten Art ausgeführt ist. Bei einer Ausgestaltung als Dental-implantat umfasst dieses erfindungsgemäß ein in einen Kieferknochen einbringbares Pfostenteil und ein diesem zugeordnetes Aufbauteil, wobei das Pfostenteil als Keramikkörper der genannten Art ausgeführt ist. Das Dentalimplantat kann dabei als so genanntes einteiliges Implantat ausgeführt sein, bei dem das Pfostenteil und das Aufbauteil Teile eines gemeinsamen, einstückig ausgeführten Grundkörpers bilden. Alternativ kann das Dentalimplantat aber auch mehrteilig in dem Sinne ausgeführt sein, dass das Pfostenteil einerseits und das Aufbauteil andererseits jeweils unabhängige Bauteile sind, die über eine geeignete Verbindung zusammengefügt werden können. Das Aufbauteil kann dabei insbesondere zur Anbringung eines Zahnersatzstückes vorgesehen sein.

Vorteilhafterweise ist das Pfostenteil dabei als Keramikkörper auf Basis von Yttrium- und/oder Aluminiumoxid-stabilisiertem Zirkonoxid ausgeführt. Um das Einwachsen in den Kieferknochen besonders zu begünstigen, ist das Pfostenteil dabei vorteilhafterweise zusätzlich mit einer besonders geeigneten Oberfläche ausgerüstet. Dazu ist vorzugsweise sein für den Kontakt mit menschlichem Gewebe vorgesehener Oberflächenbereich zumindest in einem Teilbereich mit einer nanoskopische Poren aufweisenden oder anderweitig nanoskopisch ausgeführten Struktur versehen ist, und der eine Verarmungszone auf Basis von Yttrium- und/oder Aluminiumoxid stabilisiertem Zirkonoxid mit im Vergleich zum Innenvolumen reduziertem Yttrium- bzw. Aluminiumoxid-Anteil aufweist.

Die mit der Erfindung erzielten Vorteile bestehen insbesondere darin, dass durch die Ausgestaltung des Keramikkörpers mit einer ausreichend leitfähigen Oberfläche auch ein solchermaßen ausgeführter Körper dem in der oben genannten Anmeldung beschriebenen Behandlungs- und Reinigungsverfahren unterzogen werden kann. Dabei kann auch für einen Keramikkörper, insbesondere für ein keramisch ausgeführtes Knochen- oder Dentalimplantat, mit hoher lokaler Präzision eine zuverlässige Reinigung auch lokal begrenzter Raumbereiche des jeweiligen Körpers vom Biofilm vorgenommen werden. Dabei kann zuverlässig das als besonders wirksam erkannte Reinigungskonzept der elektrolytischen Keimabtötung genutzt werden. Gerade bei nur begrenztem bakteriellen Befall des behandlungsbedürftigen Objekts kann dies besonders bedarfsgerecht erfolgen, und bei der Behandlung eines inserierten Dentalimplantats kann diese sogar vorgenommen werden, ohne dass eine Entfernung der Prothetik oder gegebenenfalls des Abutments notwendig wäre.

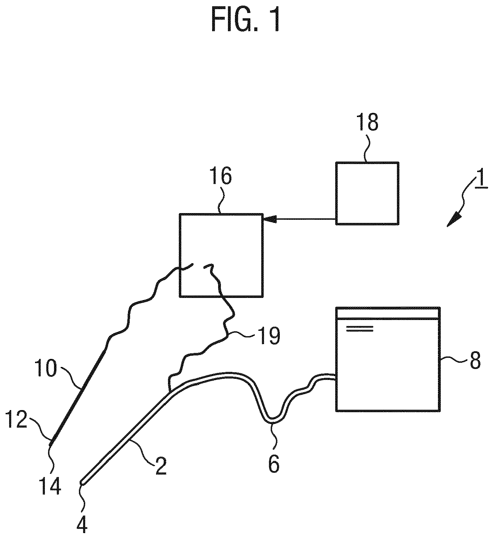

Ein Ausführungsbeispiel der Erfindung wird anhand einer Zeichnung näher erläutert. Darin zeigen:
- FIG. 1: ein Behandlungssystem zur Reinigung eines mit Biofilm verunreinigten Bauteils,
- FIG. 2: das Behandlungssystem gemäß FIG. 1 im vergrößerten Ausschnitt,
- FIG. 3: eine alternative Ausgestaltung des Behandlungssystems gemäß FIG. 1 im vergrößerten Ausschnitt, und
- FIG. 4: den Auslassbereich einer Medienkanüle im vergrößerten Ausschnitt.
Gleiche Teile sind in allen Figuren mit denselben Bezugszeichen versehen.
Das Grundprinzip der Erfindung wird nachfolgend anhand des in FIG. 1 dargestellten Behandlungssystems 1 erläutert. Selbstverständlich ist alternativ oder zusätzlich aber auch die Verwendung eines Behandlungselements möglich, wie es in der nicht vorveröffentlichten deutschen Patentanmeldung mit dem Aktenzeichen 10 2012 022 593.8, beschrieben ist.
Das Behandlungssystem 1 gemäß in FIG. 1 ist zur Reinigung eines mit Biofilm verunreinigten Bauteils, insbesondere eines Implantat-Teils, vorgesehen. Das Behandlungssystem 1 ist dabei für ein elektrolytisches Reinigungskonzept ausgelegt, bei dem das behandlungsbedürftige Bauteil gezielt und lokalisiert mit einer spezifischen, geeignet gewählten Behandlungsflüssigkeit beaufschlagt und dann ein Stromfluss durch das behandlungsbedürftige Bauteil und die Behandlungsflüssigkeit erzeugt wird. Dazu umfasst das Behandlungssystem 1 eine Medienkanüle 2, in der die Behandlungsflüssigkeit geführt und über eine Auslassöffnung 4 ausgebracht werden kann. Die Medienkanüle 2 weist dabei in ihrem Endbereich eine länglich erstreckte Bauform auf, so dass eine gezielt lokalisierte und kontrollierte Ausbringung der Behandlungsflüssigkeit möglich ist.

Medienseitig ist die Medienkanüle 2 über einen Verbindungsschlauch 6 mit einem Vorratsbehälter 8 für die Behandlungsflüssigkeit verbunden.

Zusätzlich ist das Behandlungssystem 1 noch als elektrisches System spezifisch ausgestaltet. Dabei ist als Auslegungsgrundsatz insbesondere vorgesehen, eine Beaufschlagung des in der Medienkanüle 2 geführten Mediums, insbesondere der darin geführten Behandlungsflüssigkeit, mit Strom, insbesondere mit Strompulsen, zu ermöglichen. Das Behandlungssystem 1 ist dabei dafür ausgelegt, den zu Reinigungszwecken des behandlungsbedürftigen Bauteils vorgesehenen Stromfluss gezielt lokalisiert im behandlungsbedürftigen Raumbereich aufbringen zu können. Das Behandlungssystem 1 ist dabei nach dem Auslegungsprinzip aufgebaut, dass der elektrische Strom dem behandlungsbedürftigen Bauteil zugeführt und dieses als Elektrode verwendet werden kann. Dazu umfasst das Behandlungssystem 1 ein einen elektrischen Strompfad bildendes Leiterelement 10. Dieses ist im Ausführungsbeispiel in der Art einer "konventionellen" Elektrode, also insbesondere als elektrisch leitfähiges nadelartiges Element aus Metall, ausgeführt. Das Leiterelement 10 ist dabei an seinen Außenseiten mit einer elektrischen Isolierung versehen und weist lediglich an seinem freien Ende 12 eine frei liegende metallische Kontaktspitze 14 auf. Diese kann im Betriebsfall geeignet an das behandlungsbedürftige Bauteil angedrückt und somit ein elektrischer Kontakt zu diesem hergestellt werden. Elektrisch ist das Leiterelement 10 mit einem der Pole einer elektrischen Versorgungseinheit 16, insbesondere einer Strom- oder Spannungsquelle, verbunden.

Der elektrischen Versorgungseinheit 16 ist eine Steuereinheit 18 zugeordnet, über die der gelieferte Strom bzw. die gelieferte Spannung steuer- und einstellbar sind. Die Steuereinheit wirkt dabei zusätzlich auch auf ein nicht näher dargestelltes Fördersystem des Verbindungsschlauchs 6, mit dem die Durchflussrate der Behandlungsflüssigkeit durch den Verbindungsschlauch 6 einstellbar ist.

Zur Bildung eines Gegenpols oder der Gegenelektrode ist die Nutzung der elektrischen Leitfähigkeit der in den Medienkanüle 2 geführten Behandlungsflüssigkeit vorgesehen. Dazu ist der Innenraum der Medienkanüle 2 seinerseits über ein Kabel 19, das leitend mit dem Innenraum der Medienkanüle 2 verbunden ist, elektrisch mit dem anderen Pol der elektrischen Versorgungseinheit 16 verbunden. Damit bildet die Auslassöffnung 4 der Medienkanüle 2 in elektrischer Hinsicht einen Kontakt oder elektrischen Kontaktpunkt, über den der Stromfluss in das behandlungsbedürftige Bauteil erfolgt. Bei geeigneter Positionierung der Medienkanüle 2 und ihrer Auslassöffnung 4 möglichst in unmittelbarer Nähe zum behandlungsbedürftigen Bauteil und die Nutzung der Auslassöffnung 4 als elektrischen Kontakt ist erreicht, dass der zu Behandlungs- und Reinigungszwecken angelegte elektrische Strom durch die von den Bakterien befallene Oberflächenzone des behandlungsbedürftigen Bauteils hindurch- und von dort aus weitgehend unmittelbar, also insbesondere ohne "Umwege" über weiteres Körpergewebe oder dergleichen, zur als Kontaktfläche dienenden Auslassöffnung 4 fließen kann. Die Medienkanüle 2 einschließlich der darin geführten, elektrisch leitfähigen Behandlungsflüssigkeit und den entsprechenden Anschlusselementen bildet somit im Ausführungsbeispiel ein zweites, einen elektrischen Strompfad zur Auslassöffnung 4 bildendes Leiterelement.

Die Medienkanüle 2 ist aus einem geeignet gewählten elektrisch isolierenden Grundstoff, beispielsweise aus einem Kunststoff, gefertigt. Um dabei aber die Nutzung der in der Medienkanüle 2 befindlichen Behandlungsflüssigkeit als elektrisches Leiterelement noch weiter zu begünstigen und insbesondere eine zuverlässige elektrische Kontaktierung sicherzustellen, ist die Medienkanüle 2 innenseitig, also an ihrer der Behandlungsflüssigkeit zugewandten Innenoberfläche, mit einer elektrisch leitfähigen Beschichtung versehen.

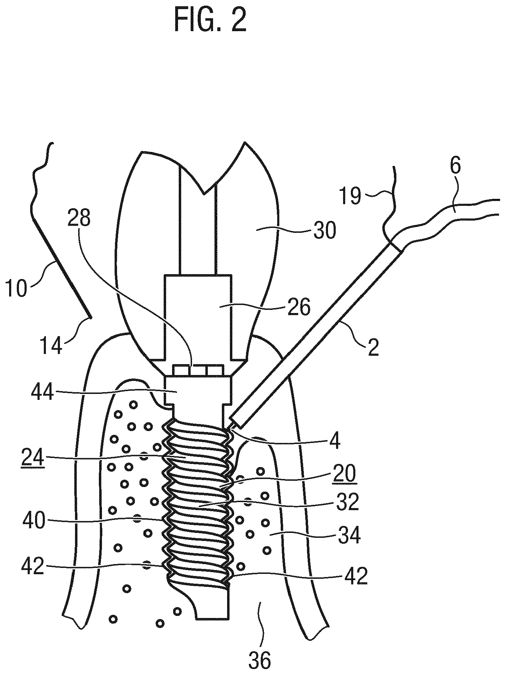

Wie der vergrößerten Darstellung gemäß FIG. 2 entnehmbar ist, ist die Medienkanüle 2 zur gezielten und lokalisierten Zuführung der Behandlungsflüssigkeit an das behandlungsbedürftige Bauteil ausgelegt. Dieses Bauteil soll sodann seinerseits als Elektrode verwendet und über das Leiterelement 10 kontaktiert werden. Allerdings handelt es sich beim behandlungsbedürftigen Bauteil um einen Keramikkörper 20, der im Ausführungsbeispiel als in den Mundknochen eines Patienten inseriertes Pfostenteil 22 eines Dentalimplantats 24 ausgeführt ist. Selbstverständlich sind aber auch andere Ausführungsformen denkbar, bei denen auf flexible und fokussierte Weise ein Keramikkörper, beispielsweise ein Knochenimplantat beliebiger Bauweise oder eine Prothetik-Komponente, beispielsweise ein Aufbauteil eines Dentalimplantats, vom Befall eines Biofilms gereinigt werden soll.

Das Dentalimplantat 24 ist im Ausführungsbeispiel zusätzlich zum Pfostenteil 22 ein Aufbauteil 26, das über eine Verbindungsschraube 28 am Pfostenteil 22 fixiert werden kann und ein Zahnersatzstück 30 trägt. Selbstverständlich könnte das Dentalimplantat 24 stattdessen auch einteilig ausgeführt sein, wobei das Pfostenteil 22 und das Aufbauteil 26 in der Art einer einstückigen Ausführung Bestandteile ein und desselben Grundkörpers wären. In FIG. 2 ebenfalls dargestellt ist ein dem Pfostenteil 22 im Bereich seines Außengewindes 32 benachbarter, räumlich begrenzter Raumbereich 34 im Kieferknochen 36, der von Periimplantitis befallen und dementsprechend mit Bakterien belastet ist.

Generell besteht bei Dental-Implantatsystemen, insbesondere auch bei zweiteiligen Implantatsystemen, das Problem, dass durch ein Eindringen von Bakterien oder Keimen in den Gewebebereich in der Nähe der Insertionsstelle, insbesondere im Bereich des in den Kiefer eingebrachten Außengewindes 32, Entzündungen oder Entzündungsherde entstehen können. Derartige, insbesondere auch in Folge einer so genannten Periimplantitis entstehende Entzündungen können, insbesondere wenn sie sich über einen längeren Zeitraum entwickeln und verfestigen können, zu einer gravierenden Beeinträchtigung des Gewebes und des Knochens im Bereich der Insertionsstelle führen. Ohne geeignete Gegenmaßnahmen können diese Beeinträchtigungen dazu führen, dass das gesamte Implantatsystem wieder aus dem Knochen entfernt und durch andere Prothetik ersetzt werden muss. Dieser durch die Periimplantitis hervorgerufene, äußerst unerwünschte Effekt kann somit zu einem Totalverlust des Implantatsystems führen, so dass erneute chirurgische Maßnahmen wie beispielsweise ein Ausschaben des betroffenen Bereichs im Kieferknochen und die Neuversorgung mit einem Implantatsystem notwendig werden können. Durch eine derartige Entnahme kann es zudem zu Knochenverlust oder sonstigem Verlust an Gewebesubstanz kommen, die im Extremfall so weit führen kann, dass eine Neuversorgung mit einem anderen Implantat gar nicht mehr möglich ist. Eine derartige durch Periimplantitis hervorgerufene Notwendigkeit einer Neuversorgung kann auch nach vergleichsweise langen Zeiträumen nach dem ersten Einsetzen des Implantatsystems von beispielsweise bis zu einigen Jahren oder sogar Jahrzehnten auftreten.

Die im Zusammenhang mit einer Periimplantitis beobachteten Keime oder Bakterien können dabei grundsätzlich das Innere der Komponenten des Dentalimplantats 24 besiedeln, haften aber in der Regel bevorzugt direkt an der Oberfläche des in den Kieferknochen 36 inserierten Pfostenteils 22 im Kontaktbereich mit dem umgebenden Gewebe oder Knochenmaterial, also insbesondere im Bereich des Außengewindes 32, an. In dessen Bereich kann die Oberfläche des Pfostenteils 22 mit einer Aufrauhung oder dergleichen versehen sein, um das Einwachsen in das Gewebe oder den Knochen besonders zu begünstigen und das Einheilen des Pfostenteils 22 nach der Insertion zu unterstützen. Gerade im Bereich einer derartigen, eigentlich als besonders günstig für das Implantatsystem angesehenen Aufrauhung der Oberfläche kann aber die Ansiedlung der Keime oder Bakterien vermehrt stattfinden, wobei die Rauigkeit ein gezieltes Entfernen der vorhandenen Keime oder Bakterien noch zusätzlich erschwert.

Es besteht daher der dringende Wunsch nach geeigneten Gegenmaßnahmen, um für den Fall einer sich anbahnenden oder bereits aufgetretenen Periimplantitis unter Erhaltung des bereits eingesetzten Implantatsystems wirksam den Entzündungsherd bekämpfen und die eingedrungenen Keime abtöten zu können, so dass sich anschließend wieder gesundes Gewebe oder gesunde Knochensubstanz im Bereich um das Außengewinde 32 herum bilden kann. Dazu ist wünschenswert, zusätzlich zu einem gezielten Abtöten der Keime oder Bakterien im betroffenen Bereich auch noch deren Materialreste und Fragmente zuverlässig aus dem betroffenen Raumbereich zu entfernen, so dass anschließend der betroffene Bereich wieder von gesundem Gewebe oder Knochenmaterial ausgefüllt werden und sich wieder eine innige Verbindung zwischen der Außenoberfläche des Pfostenteils 22 und dem umgebenden Gewebe oder Knochenmaterial bilden kann. Zudem sollte der von der Bakterienbeschichtung gebildete Biofilm einschließlich der organischen Reste abgetöteter Bakterien zuverlässig entfernt werden.

Zu diesem Zweck, also zum Abtöten von Keimen oder Bakterien im Insertionsbereich des Pfostenteils 22 und insbesondere auch zum anschließenden Ausspülen, Entfernen und Austragen der Gewebe- und Materialreste der abgetöteten Bakterien, ist das Behandlungssystem 1 vorgesehen. Dieses beruht hinsichtlich seiner Ausgestaltung und prinzipiellen Ausführung auf zwei jeweils als eigenständig erfinderisch angesehenen Grundkonzepten: Einerseits ist es dafür ausgestaltet, die im Insertionsbereich des Dentalimplantats 24 vorhandenen Keime oder Bakterien durch gezielte Zuführung eines bakterioziden, aber für den menschlichen Organismus verträglichen Reinigungs- oder Desinfektionsmittels gezielt abzutöten. Andererseits ist es dafür ausgelegt, eventuell an der Oberfläche des Pfostenteils 22, insbesondere im Bereich des Außengewindes 32, noch anhaftende Reste oder Fragmente von Keimen und/oder Bakterien durch eine geeignete Beaufschlagung mit Strom oder Stromstößen von der Außenoberfläche des Pfostenteils 22 abzulösen, so dass sie anschließend ausgewaschen werden können.

In einem ersten, sowohl bezüglich der Ausgestaltung des Systems als auch bezüglich der vorgesehenen Verfahrensschritte des Behandlungsverfahrens eigenständig als erfinderisch angesehenen Aspekt ist das Behandlungssystem 1 daher sowohl strukturell als auch funktional/konzeptionell dafür ausgelegt, die Behandlungsflüssigkeit zum Abtöten der Keime oder Bakterien und/oder zum Reinigen des inserierten Implantat-Teils gezielt in den Insertionsbereich des Pfostenteils 22, insbesondere den Bereich von dessen Außengewinde 32, einzuspeisen.

In einem zweiten, ebenfalls sowohl bezüglich der Ausgestaltung des Systems und der Auswahl und Zusammensetzung der Grundbestandteile der verwendeten Behandlungsflüssigkeit als auch bezüglich der vorgesehenen Verfahrensschritte des Behandlungsverfahrens eigenständig erfinderischen Aspekt ist das Behandlungssystem 1 dafür ausgelegt, die abgetöteten Bakterien oder Keime bzw. deren Reste oder Fragmente zuverlässig von der Außenoberfläche des Pfostenteils 22 abzulösen, so dass sie anschließend ausgespült werden können und sich nachfolgend gesundes Gewebe oder Knochenmaterial an die Oberfläche des Pfostenteils 22 anlegen und dieses wieder vollständig in gesundes Gewebe oder Knochenmaterial einwachsen kann. Für die Ablösung der Bakterien oder Keime bzw. deren Resten oder Fragmenten von der Oberfläche ist deren Benetzung mit einer leitfähigen Behandlungsflüssigkeit unter Beaufschlagung mit Strom vorgesehen. Wie sich nämlich ebenfalls völlig überraschend herausgestellt hat, scheint gerade diese Beaufschlagung mit Strom in Kombination mit geeignet gewählten lonenkonzentrationen in der Behandlungsflüssigkeit besonders zuverlässig das Ablösen der Bakterien oder Keime bzw. von deren Fragmenten oder Resten von der darunterliegenden Oberfläche zu bewirken, selbst wenn diese aufgeraut ist und eigentlich das Anhaften organischen Materials auf Grund ihrer Oberflächenstruktur besonders begünstigt.

Dabei liegt die überraschende Erkenntnis zugrunde, dass die Beaufschlagung des Pfostenteils 22 mit Strom unter Verwendung einer geeignet gewählten Behandlungsflüssigkeit im Bereich der Außenoberfläche des Pfostenteils, also insbesondere im Bereich des Außengewindes 32, zu einer elektrolytischen Reaktion in der Behandlungsflüssigkeit und damit gegebenenfalls zur Erzeugung von Gasblasen in unmittelbarer Nähe zur Oberfläche führt. Durch diese Gasblasenbildung an der Oberfläche des Pfostenteils 22 werden die oberflächlich anhaftenden Bestandteile oder Fragmente der Keime oder Bakterien mit abgelöst und restlos entfernt, so dass sie keine Basis und keinen Nährboden für eine Neuansiedlung von Keimen in diesen Bereichen bilden können. Zurück bleibt eine von Keimen, Bakterien oder deren Bestandteilen oder Resten befreite, angeraute und poröse Oberfläche des Pfostenteils 22, die gut als Basis für eine zukünftige Integration in das nachwachsende Knochengewebe dienen kann.

Allerdings ist für ein derartiges Behandlungskonzept erforderlich, dass das Pfostenteil 22 - oder das behandlungsbedürftige Bauteil im Allgemeinen - als Elektrode in dem Behandlungssystem 1 genutzt werden kann. Hierfür ist der das Pfostenteil 22 bildende Keramikkörper 20 gezielt geeignet ausgelegt. Um dies zu ermöglichen, weist der Keramikkörper zur 20 in seinem für den Kontakt mit menschlichem Gewebe vorgesehenen Oberflächenbereich 40 eine ausreichend hohe elektrische Leitfähigkeit und somit einen ausreichend niedrigen spezifischen elektrischen Widerstand von höchstens 10⁻² Ω cm, vorzugsweise von höchstens 10⁻⁵ Ω cm, auf.

Um diese elektrische Leitfähigkeit gezielt bereitstellen zu können, kommen mehrere Ausführungsformen für den Keramikkörper 20 in Betracht. Einerseits könnte das komplette Dentalimplantat 24 oder zumindest dessen Pfostenteil 22 vollständig aus einer elektrisch leitfähigen bzw. einer elektrisch halbleitenden Keramik gefertigt sein. Dies kann dann entweder von okklusal oder über das Innere elektrisch kontaktiert werden zum Zwecke der Reinigung. Eine elektrische Kontaktierung über die Medienkanüle 2 von außen kann selbstverständlich auch angewendet werden.

Alternativ ist auch denkbar, dass einer nicht elektrisch leitfähigen Keramik, welche sich als Implantatmodul eignet, ein elektrisch leitender oder halbleitender Stoff, vorzugsweise auf keramischer und/oder Kohlenstoffbasis beigemischt wird, um eine elektrische Leitfähigkeit oder elektrische Halbleitfähigkeit hervorzurufen.

Weiterhin alternativ kann das Dentalimplantat 24 bzw. dessen Pfostenteil 22 als vollkeramischer, elektrisch nicht leitfähiger Keramikkörper 20 ausgeführt sein, wie dies im Ausführungsbeispiel auch vorgesehen ist. Um die erwünschte elektrische Leitfähigkeit im Oberflächenbereich 40 dennoch sicherzustellen, ist dieser mit einer elektrischen leitfähigen bzw. elektrisch halbleitenden Beschichtung 42 versehen. Diese Beschichtung 42 kann vollständig/gänzlich oder nur in Teilbereichen der Oberfläche des Pfostenteils 22 vorgesehen sein und ist im gezeigten Ausführungsbeispiel lediglich am Pfostenteil 22 angebracht. Selbstverständlich ist es - je nach vorgesehenem Einsatzzweck - aber auch möglich, andere Bauteile wie beispielsweise das als Prothetik-Komponente vorgesehene Aufbauteil 26 mit ganz oder teilweise mit der Beschichtung 42 zu versehen. Zur elektrischen Kontaktierung ist im Ausführungsbeispiel die Implantatschulter 44 vorgesehen, in deren Bereich die Beschichtung 42 eine erhöhte Dicke, z. B. doppelt, fünffach, zehnfach so dick wie die Beschichtung 42 im sonstigen Oberflächenbereich 40, aufweist. Würde man das Äußere und das Innere eines Implantats solchermaßen beschichten, wäre auch eine elektrische Kontaktierung über das Innere des Implantats möglich.

Das Pfostenteil 22 ist als Keramikkörper 20 auf Basis von Yttrium-und/oder Aluminiumoxid-stabilisiertem Zirkonoxid ausgeführt. Sein für den Kontakt mit menschlichem Gewebe vorgesehener Oberflächenbereich 40 ist zudem zumindest in einem Teilbereich mit einer nanoskopische Poren aufweisenden oder anderweitig nanoskopisch ausgeführten Struktur versehen und weist eine Verarmungszone auf Basis von Yttrium- und/ oder Aluminiumoxid stabilisiertem Zirkonoxid mit im Vergleich zum Innenvolumen reduziertem Yttrium- bzw. Aluminiumoxid-Anteil auf.

Die Beschichtung 42 kann dabei von einem Metall, von einem elektrisch leitfähigen Kunststoff und/oder von einem elektrisch leitfähigen Kohlenstoff gebildet sein. Im Ausführungsbeispiel ist die Beschichtung 42 jedoch von einer dotierten Keramik, vorzugsweise von Indiumoxid und/oder Indium-Zinn-Oxid, von Zinkoxid, von Siliziumnitrit, von MoSi₂, von Si₃N₄-MoSi₂-SiC, von ZrO₂/CaO, von Al₂O₃/TiN, von BaTiO₃, von einem Silizid, von einem Nitrid, von einer titanhaltigen Keramik und/oder von einem Titanat gebildet.

Eine derartige elektrisch leitfähige Beschichtung 42 kann selbst bei an sich metallischen Dentalimplantaten vorteilhaft sein. Bei der anodischen Beschaltung von Titanimplantaten, insbesondere von Titan Grade IV oder reinerem Titan ergibt sich nämlich eine Titanoxidschichtbildung, welche direkt nach ihrer Entstehung ohne Erhöhung der elektrischen Spannung die elektrochemischen Prozesse an der Oberfläche sehr stark reduziert bzw. unterbindet. Eine anodische Bestromung zur Reinigung ist somit nur sehr kurzfristig bzw./oder einmalig möglich, ohne den Patienten durch die elektrische Spannung zu gefährden. Würde man ein solches Titanimplantat mit einer elektrisch leitfähigen bzw. elektrisch halbleitenden Keramik beschichten, könnte man das Titanoxidwachstum bei anodischer Bestromung verhindern. Auf diese Weise könnte man es ermöglichen, die anodische Bestromung zur Reinigung der Implantatoberfläche lang dauernd zu nutzen. Als weiterer Vorteil einer solchen Oberfläche würde es sich ergeben, dass keine Metallionen aus dem metallischen Implantat herausgelöst werden könnten.

Eine besondere Begünstigung der im Sinne einer zuverlässigen Reinigung der Oberfläche erwünschten Ablösung der an der Oberflächen haftenden Biofilmbestandteile aus dem inserierten Pfostenteil 22 ist durch eine vorteilhafte besonders geeignete Verfahrensführung bei der Beaufschlagung mit Strom erreichbar. Diese kann derart vorgenommen werden, dass die infolge des Stromflusses im Bereich der inserierten Oberfläche stattfindende elektrolytische Gasblasenbildung besonders verstärkt wird. Hierbei kann das Pfostenteil 22 anodisch oder auch kathodisch geschaltet werden. Insbesondere bei einer zumindest vorübergehend kathodischen Beschaltung des Pfostenteils 22 entsteht elektrolytisch induziert Wasserstoffgas, das besonders wirksam zur Gasblasenbildung beiträgt. Bei anodischer Schaltung des Pfostenteils entstehen hingegen, abhängig von der Zusammensetzung der Behandlungsflüssigkeit, Chlorgas, Sauerstoff, Stickstoff; Kohlenmonoxid und/oder Kohlendioxid. Die dadurch gebildeten Gasblasen steigen in der umgebenden Flüssigkeit auf und erzeugen dadurch Mitnahmeeffekte, über die die genannten Oberflächenbestandteile mit abgetragen und nach außen hin abgefördert werden. Beispielsweise wurde völlig überraschend beobachtet, dass bei Verwendung einer positive Ionen enthaltenden Lösung, beispielsweise einer wässrigen Salzlösung, diese Ionen sich bei einer kathodischen Beschaltung des Pfostenteils 22 an diesem abscheiden und damit die Gasblasenbildung deutlich verstärken. Beispielsweise führt die Anwesenheit von Na⁺-Ionen bei kathodischer Verschaltung des Pfostenteils 22 zu erheblicher Gasblasenbildung, da Na+ mit dem umgebenden Wasser zu NaOH reagiert und dabei Wasserstoff freisetzt.

In einem weiteren, ebenfalls sowohl bezüglich der Ausgestaltung des Systems als auch bezüglich der vorgesehenen Verfahrensschritte des Behandlungsverfahrens eigenständig erfinderischen Aspekt ist das Behandlungssystem 1 für eine besonders einfach und effizient gehaltene Kombination der genannten Aspekte ausgestaltet. Dabei liegt das Konzept zugrunde, dass sowohl die vorgesehene Zuführung der Reinigungsflüssigkeit als auch das gezielte Ablösen der Bakterien- und Keimreste und -fragmente durch Aufbringung der genannten Strompulse in einem gemeinsamen System und somit mit besonders einfach gehaltenen Mitteln erreicht werden kann.

Die verwendete Behandlungsflüssigkeit ist im Hinblick auf diese Aspekte geeignet gewählt und zusammengesetzt. Die Auswahl und Zusammensetzung der Grundbestandteile der Behandlungsflüssigkeit ist dabei insbesondere im Hinblick auf die beabsichtigte Wirkungsweise, d. h. der Aufbringung eines elektrischen Stroms im Raumbereich der behandlungsbedürftigen Oberfläche, vorgenommen, wobei insbesondere sichergestellt ist, dass eine für diesen Zweck ausreichend hohe elektrische Leitfähigkeit in der Behandlungsflüssigkeit vorliegt. Diese soll insbesondere durch eine ausreichend hoch gewählte Ionendichte in der Behandlungsflüssigkeit sichergestellt werden. Dazu ist als ein Grundbestandteil der Behandlungsflüssigkeit ein Metallsalz vorgesehen, bevorzugt in wässriger Lösung. Dieses liefert die Ionen für den Stromtransport, und zudem können die nach der jeweiligen Elektrodenreaktion entstehenden Umsetzungsprodukte auch noch geeignete biochemische Wirkungen aufweisen. Durch die gezielte Wahl einer ausreichend hohen elektrischen Leitfähigkeit soll bei einer Durchführung des Reinigungsverfahrens an einem inserierten Implantat sichergestellt werden, dass der Stromfluss durch die Behandlungsflüssigkeit und damit durch die behandlungsbedürftigen Teile und Komponenten, nicht aber durch das Körpergewebe des Patienten erfolgt, so dass eine Gefährdung des Patienten durch einen ungewollten Stromfluss durch Weichgewebe, Knochen, Blut und/oder andere Körpermaterialien minimiert werden kann. Die elektrische Leitfähigkeit der Behandlungsflüssigkeit sollte dabei möglichst ein Vielfaches der elektrischen Leitfähigkeit von Blut, Knochen, Weichgewebe, Fettgewebe oder anderen Körpermaterialien aufweisen.

Demzufolge werden bei der Auswahl und Zusammensetzung der Grundbestandteile für die Behandlungsflüssigkeit insbesondere folgende Leitfähigkeitswerte berücksichtigt (die elektrische Leitfähigkeit σ wird dabei in der üblichen Einheit mS/cm angegeben):

| | |
|---|---|
| Haut: | 0,03 - 0.1 mS/cm |
| Knochen: | 0,06 - 0,2 mS/cm |
| Fettgewebe: | 0,20 - 1,0 mS/cm |
| Muskelgewebe: | 0,80 - 2,5 mS/cm |
| Blut: | ca. 6,7 mS/cm |
| andere Körperflüssigkeiten: | ca. 15 mS/cm |

Um das Gefährdungspotential für den Patienten geeignet gering zu halten und den Stromfluss auf die erwünschten Regionen zu begrenzen, sollte die elektrische Leitfähigkeit daher mindestens das Doppelte, bevorzugt das Fünffache, besonders bevorzugt das Zehnfache der Leitfähigkeit sonstiger Körperflüssigkeiten betragen. Daher sollte die elektrische Leitfähigkeit der Behandlungsflüssigkeit einen Wert von mindestens 30 mS/cm, vorzugsweise mindestens 75 mS/cm und besonders bevorzugt mindestens 150 mS/cm aufweisen. Im Vergleich zu Blut bedeutet dies, dass die elektrische Leitfähigkeit der Behandlungsflüssigkeit vorzugsweise mindestens etwa das Fünffache, bevorzugt mindestens etwas das Zehnfache und besonders bevorzugt mindestens etwa das Zwanzigfache der Leitfähigkeit von Blut beträgt. Messungen haben ergeben, dass beim Einsatz einer solchermaßen gewählten Behandlungsflüssigkeit die elektrische Spannung, der das Körpergewebe, das Blut, die Körperflüssigkeiten etc. ausgesetzt werden, geringer als 6 V, vorzugsweise geringer als 3 V, besonders bevorzugt kleiner als 1,5 V ist. Damit können Schädigungen für den Patienten aufgrund der gering gehaltenen Spannungen sicher ausgeschlossen werden. Zur Einhaltung einer solchen Leitfähigkeit ist insbesondere die lonenkonzentration in der Behandlungsflüssigkeit und in den sie bildenden Grundbestandteilen ausreichend hoch gewählt; hierfür können Laugen, Säuren, Salze und/oder andere ionenbildende Stoffe oder Stoffverbindungen zum Einsatz kommen.

Bei der Auswahl und Zusammensetzung der Grundbestandteile der Behandlungsflüssigkeit ist in besonderem Maße berücksichtigt, dass die reinigende oder biofilmablösende Wirkung der elektrolytischen Behandlung einer kontaminierten Implantatoberfläche auf einer Kombination mehrerer Ursachen beruht, die möglichst ergänzend zueinander nutzbar gemacht werden sollten. Zum einen können sich beim Stromfluss durch den Elektrolyten bevorzugt im Bereich der Elektroden Gase oder Gasblasen bilden, welche eine abhebende (mechanische) Wirkung auf den Biofilm haben. Die Entstehung dieser Gase erfolgt unmittelbar an der als Elektrode dienenden Implantatoberfläche und somit zwischen dieser und dem Biofilm. Die dabei entstehenden Gasblasen beeinflussen mit ihrer Wachstumsgeschwindigkeit und ihrer maximalen Größe den Ablösungsprozess.

Als zweite Ursache für die das Implantat reinigende oder den Biofilm ablösende Wirkung des elektrolytischen Prozesses ist die zersetzende, zerstörende und auflösende Wirkung der elektrolytisch entstehenden Stoffe oder Stoffverbindungen auf die eigentliche Anhaftung des Biofilms an der Implantatoberfläche, also auf den Klebe- oder Verankerungsmechanismus, zu nennen.

Die dritte Ursache für die reinigende oder ablösende Wirkung des elektrolytischen Prozesses basiert auf materialabtragenden Effekten des Implantatmaterials, wobei Bestandteile oder Partikel des eigentlichen Implantats in dessen Oberflächenbereich aus diesem herausgelöst werden.

Die vierte Ursache für die reinigende oder ablösende Wirkung des elektrolytischen Prozesses basiert auf der Oxidschichtbildung metallischer Implantate, welche dies erlauben. Hierbei durchdringen Metallatome des metallischen Grundmaterials die evtl. schon vorhandene Oxidschicht basierend auf der angelegten elektrischen Spannung und reagieren mit Stoffen des Elektrolyts (meist Sauerstoff => Metalloxidbildung). Bei Metallen, welche keine Oxidschicht bzw. keine mechanisch stabile Oxidschicht bilden, können auch nicht-oxydische Stoffverbindungen entstehen (meist Salze), die dann in Lösung gehen.

Die für die Bildung der Behandlungsflüssigkeit vorgesehenen Grundbestandteile sind im Hinblick auf diese Effekte geeignet gewählt und miteinander kombiniert. Zudem ist als grundlegendes Auslegungsziel berücksichtigt, dass keine toxischen oder anderweitig einen Patienten gefährdenden oder für ihn unangenehmen Effekte auftreten sollten, so dass die Behandlungsflüssigkeit auch für einen Einsatz am inserierten Dentalimplantat, also im Patientenmund, geeignet ist. Im Ausführungsbeispiel sind dabei als Grundbestandteile mindestens ein Salz einerseits und eine Säure andererseits, bevorzugt verdünnt mit Wasser, vorgesehen, deren Auswahl und Zusammensetzung sich insbesondere nach den genannten Kriterien richtet. Besonders bevorzugt ist dabei als Säure Phosphorsäure, Zitronensäure, Ameisensäure, Essigsäure, Milchsäure, Kohlensäure oder eine Kombination von diesen vorgesehen. Alternativ oder zusätzlich besonders bevorzugt ist dabei als Salz Natrium-, Calcium-, Aluminium-, Magnesium-, Zinn- oder Kaliumiodid, -chlorid, -nitrat, -carbonat oder -hydrogencarbonat und/oder Ammoniumchlorit, -nitrat oder -iodid oder eine Kombination von diesen vorgesehen.

Das Pfostenteil 22 ist bei der Behandlung mit der Behandlungsflüssigkeit bevorzugt kathodisch geschaltet. Dabei wandern positiv geladene Ionen (Kationen) zur Oberfläche des Pfostenteils 22. Dies können insbesondere H⁺-Ionen, Metallionen oder langkettige Kohlenwasserstoffionen, z. B. aus ionischen Flüssigkeiten. Das als Grundbestandteil für die Behandlungsflüssigkeit vorgesehene Salz ist dabei insbesondere gezielt im Hinblick auf die Eigenschaften der Kationen ausgewählt, die den genannten Prozess begünstigen oder überhaupt ermöglichen sollen. Zur Erzeugung einer möglichst hohen elektrischen Leitfähigkeit eignen sich insbesondere kleine Ionen (H⁺-Ionen oder Metallkationen), die zudem in der Art eines weiteren besonders günstigen Effekts den gegebenenfalls vorhandenen Biofilm vergleichsweise leicht durchdringen können. H⁺-Ionen werden an der durch das Pfostenteil 22 gebildeten Kathode zu elementarem Wasserstoff H reduziert. Dies erzeugt eine Blasenbildung.

Alkalimetalle, Erdalkalimetalle und/oder Aluminium reagieren an der Kathode mit dem umgebenden Wasser zu elementarem Wasserstoff und seinem Metallkationen und OH⁻-Ionen. Dies bedeutet, dass sich Wasserstoffblasen und das Hydroxid des verwendeten Metallions bilden. Durch die Kombination dieser Komponenten ist somit neben der ablösenden Wirkung des entstehenden Wasserstoffs erreicht, dass das Metallhydroxid antibakteriell wirkt und einen verdünnenden oder auflösenden Einfluss auf den Biofilm bzw. dessen Adhäsionsmechanismus ausübt.

Um Unverträglichkeiten mit dem Körpergewebe zu vermeiden, sind als Metallkationen insbesondere die körpereigenen (z. B. Kalium- und/oder Natriumionen) besonders bevorzugt. Darüber hinaus eignen sich auch Calcium, Magnesium und/oder Aluminiumionen. Das als Grundbestandteil für die Behandlungsflüssigkeit vorgesehene Salz ist daher besonders bevorzugt ein Salz dieser Metalle, insbesondere da diese Metallkationen ohnehin nur in Form eines Salzes, z. B. in Wasser gelöst, zur Verfügung gestellt werden können.

Diese Metallsalze können Verbindungen der genannten Metalle mit einem geeigneten Salzbildner, beispielsweise mit Schwefel, Phosphor, Stickstoff, Fluor, Chlor, Jod, Brom, Kohlenwasserstoff, Sauerstoff, Bor oder anderen Nichtmetallen, sein. Der Salzbildner wird dabei vorteilhafterweise unter Berücksichtigung des Grundsatzes "je größer das Anion, desto geringer die elektrische Leitfähigkeit" und im Hinblick auf die grundsätzlich gewünschte hohe elektrische Leitfähigkeit geeignet gewählt. Als Anion werden zudem bevorzugt nur Stoffe in Betracht gezogen, welche weder die Gesundheit noch das periimplantäre Gewebe beeinflussen. Weiterhin gilt zu beachten, dass unangenehme Gerüche oder Geschmacksverbindungen unerwünscht sind. Aus diesen Gründen werden Schwefelanionen oder Anionen, die Schwefel in Kombination mit Sauerstoff oder anderen Elementen enthalten, als eher ungeeignet angesehen. Dies gilt auch für Fluor-, Brom-, Stickstoff- und Borionen, gegebenenfalls auch in Kombination mit weiteren Elementen.

Demgegenüber haben Phosphate, Phosphationen und Hydrogenphosphationen meist keine oder kaum eine schädliche Wirkung. Chlorionen oder Ionen, welche Chlor enthalten, haben meist eine antibakterielle Wirkung. Sollte das Chlorion allerdings elektrolytisch oxidiert werden und in Wasser elementar vorhanden sein, so bildet sich dabei Salzsäure und hypochlorige Säure. Dies würde zwar in Kombination mit dem kathodisch entstandenen Hydroxid zu einer Neutralisierung führen. Jedoch hat sich bei Untersuchungen gezeigt, dass das Chlor, welches an der Gegenelektrode zum Implantat (Anode) entsteht, in großem Maße dem Elektrolyten als Gas entweicht. Sollte das Chlor bei der Behandlung nicht restlos abgesaugt werden können, kann es zu starken Verätzungen in der Lunge und/oder den Schleimhäuten kommen. Hierbei gilt es abzuwägen, ob der Nutzen für den Patienten oder dessen Gefährdung größer ist.

Zu den Phosphaten des Aluminiums, des Kaliums, des Natriums, des Calciums oder des Magnesiums ist zudem zu bemerken, dass die Löslichkeit in Wasser so gering ist, dass eine ausreichende elektrische Leitfähigkeit des Elektrolyten nicht gewährleistet ist (diese Phosphate eignen sich allerdings sehr gut als Zusatzstoffe des Elektrolyten zur Pufferung des pH-Wertes). Chloride der vier eben aufgeführten Metalle hätten zwar eine ausreichende Löslichkeit in Wasser und eine gute Reinigungs- und Abtötungswirkung auf den Biofilm, können aber nicht als das Optimum angesehen werden. Bei Nitraten und/oder Nitriten ist eine Gefährdung des Patienten durch die Bildung von NOₓ-Gasen zu erwarten. Aus diesem Grund ist vom Einsatz von Nitriten oder Nitraten abzuraten.

Im Hinblick auf die genannten Auslegungsziele, insbesondere für eine besonders gute Verträglichkeit für den Patienten, ist in bevorzugter Ausführung als Salzbildner Jod vorgesehen. Besonders vorteilhaft ist dabei, dass Jodsalze des Kaliums und des Natriums auch im menschlichen Körper von Natur aus vorhanden sind. Bei der Oxidation von Jodionen an der Anode entsteht zunächst elementares Jod, welches sich in eine Natriumiodid-/Kaliumiodidlösung lösen kann. Es entsteht dabei eine Jod-Kalium-Jodid-Lösung bzw. eine Jod-Natrium-Jodid-Lösung. Beide Lösungen sind starke Desinfektionsmittel, welche sich in der Humanmedizin bewährt haben.

Reine Natrium- oder Kaliumiodidlösungen oder ein Gemisch aus beiden haben jedoch als möglichen Nachteil die Natrium- und/oder Kaliumhydroxidbildung und den damit verbundenen pH-Wertanstieg zur Folge. Als problematisch könnte nämlich ganz allgemein bei der bereits oben beschriebenen Bildung von Metallhydroxid einzustufen sein, dass ein Metallhydroxid den pH-Wert des Elektrolyten erhöht. Ein solchermaßen erhöhter pH-Wert und die sich bildende Lauge oder Base des gelösten Metallhydroxids könnten einen unerwünschten Einfluss auf das umliegende Gewebe im Patientenmund und insbesondere den Knochen haben. Auch könnten umliegende Zähne geschädigt werden. Zudem könnte die Bildung von Hydroxiden dazu führen, dass diese sich aufgrund ihrer sehr geringen Wasserlöslichkeit auf dem Pfostenteil 22 oder allgemein auf dem behandlungsbedürftigen Bauteil niederschlagen und damit den weiteren Stromfluss und somit den Prozess insgesamt behindern. Allenfalls bei Verwendung eines Calciumsalzes in der Behandlungsflüssigkeit würde das entstehende Calciumhydroxid, das als Bestandteil des Knochenmaterials vorkommt, in den Knochen integriert werden können; Calcium ist somit besonders bevorzugter Bestandteil des Salzes. Um diese unerwünschten Einflüsse zu kompensieren, weist die Behandlungsflüssigkeit als weiteren Grundbestandteil in der Art eines pH-Puffers oder -Reduzierers die Säure auf.

Die Säure ist dabei ihrerseits gezielt in der Art eines Auslegungskriteriums derart gewählt, dass sie möglichst nicht den Patienten oder das periimplantäre Gewebe gefährdet, sondern zum einen das Hydroxid neutralisiert (und auch den pH-Wert möglichst nicht über 7 steigen lässt), wobei zum anderen die Reaktionsprodukte dem eigentlichen Ziel der Reinigung des Implantatkörpers und der Ablösung des Biofilms dienen sollten. Als Mineralsäuren kommen dazu bevorzugt Phosphorsäuren und/oder Phosphatsäuren in Frage. Diese sind in ihrer Konzentration aus gesundheitsgefährdenden und/oder knochen-/gewebegefährdenden Gründen limitiert. Eine besonders bevorzugte Säure, welche auch als Mineralsäure angesehen wird und einen besonders positiven Effekt auf das Gesamtziel der Abtötung und Reinigung hat, ist hingegen die Kohlensäure. Diese ist jedoch in ihrer anwendbaren Menge durch das vergleichsweise geringe Lösungsvermögen in Wasser limitiert.

Organische Säuren stellen demgegenüber ähnlich wie Mineralsäuren pH-Wert-reduzierende und Hydroxid neutralisierende H⁺-Ionen zur Verfügung. Da sie zudem keine oder allenfalls geringfügige Schädigungen im Gewebe erzeugen oder beim Patienten insgesamt hervorrufen, sind derartige organische Säuren als Grundbestandteil für die Behandlungsflüssigkeit ganz besonders bevorzugt. Organische Säuren wären z. B. Alkansäuren, Fruchtsäuren, Carbonsäuren sowie Hydroxylcarbonsäuren. Als besonders geeignete Säuren haben sich α-Hydroxylcarbonsäuren herausgestellt. Insbesondere zeigen die besonders bevorzugten Säuren Milchsäure, Zitronensäure und Apfelsäure keinerlei gesundheitsschädigende Effekte auf den Patienten generell oder auf das periimplantäre Gewebe. Speziell bei stark mit Biofilm behafteten und kontaminierten Implantaten, auf welchen sich auch Zahnstein gebildet hat, zeigten bereits vergleichsweise geringe Dosierungen an Essigsäure einen guten Reinigungserfolg. Weitere Säuren, welche die Reinigung sowie den bakterientötenden Effekt aufweisen, aber aus gesundheitlichen Gründen nicht unbedenklich sind, wären die Fumarsäure, Gluconsäure, Glycolsäure, Salicylsäure, Mandelsäure, Weinsäure, Essigsäure, Oxalsäure und Ameisensäure.

Bei der Neutralisierung des Hydroxidions OH⁻ mit dem entsprechenden H⁺-Ion einer Säure entsteht zusätzlich das Metallsalz der verwendeten Säure des entsprechenden Metallhydroxids. Der vorgesehene Einsatz der Säure ist somit nicht nur zur Pufferung des pH-Werts vorteilhaft, sondern trägt überdies noch zur Umwandlung des vergleichsweise schlecht wasserlöslichen Hydroxids in vergleichsweise gute wasserlösliche Salze bei und verhindert somit die Abscheidung unerwünschten und für den Prozess hinderlichen Niederschlags auf dem behandlungsbedürftigen Bauteil. Die genannten Salze werden insbesondere bei der Kombination der genannten bevorzugten Materielaien u. a. auch in der Medizin eingesetzt. Bei der Neutralisation des Kalium-, Natrium- und/oder Calciumhydroxids mit Milchsäure bildet sich das Kaliumlaktat (es besitzt eine breitbandige antimikrobielle Wirkung), Natriumlaktat bzw. Calciumlactat. Werden die sich bildenden Hydroxide hingegen mit Zitronensäure neutralisiert, bilden sich Zitrate des Kaliums, Natriums bzw. Calciums. Gerade für Natriumzitrat ist dies besonders vorteilhaft, da dieses die Blutgerinnung verhindert. Dies ist besonders vorteilhaft, da während des Prozesses austretendes, auf der Implantatoberfläche gerinnendes Blut die Ionenwanderung zur Implantatoberfläche und damit die Fortsetzung des Behandlungsprozesses insgesamt behindern könnte.

Bei der Neutralisation der Hydroxide mit Apfelsäure entstehen hingegen Malate des jeweiligen Kations, welche ebenfalls günstige Auswirkungen auf den Prozess haben. Bei der Neutralisation der Hydroxide mit Essigsäure entstehen Acetate des Kaliums, Natriums und/oder Calciums, welche ebenfalls einen günstigen Einfluss auf den Prozess haben.

Die Lactate, Zitrate, Malate und/oder Acetate des Kaliums, Natriums und/oder Calciums besitzen alle eine säureregulierende Wirkung und sind so verträglich, dass sie nach den jetzigen EU-Verordnungen für Lebensmittelzusatzstoffe in ihrer Verwendung an keine Mengenlimitierung gebunden sind.

Bei der Verwendung von Säuren in dem Elektrolyten in Kombination mit Jodiden und/oder Chloriden des Natriums, Kaliums, Magnesiums, Aluminiums und/oder Calciums hat sich bei der elektrolytischen Anwendung überraschend herausgestellt, dass durch die direkte Reduzierung des H⁺-Ionen die Blasenbildung derart positiv beeinflusst wird, dass sich der Biofilm deutlich schneller und besser ablöst. Dabei entsteht mit hoher Erzeugungsrate eine Vielzahl vergleichsweise kleiner Bläschen, die aufgrund ihrer vergleichsweise geringen Größe den Biofilm als Ganzes und nicht nur lokal von der darunterliegenden Oberfläche lösen können. Dadurch wird der Biofilm bevorzugt als Ganzes oder in vergleichsweise großen, zusammenhängenden Stücken statt in einer Vielzahl kleinerer Fragmente abgehoben, was eine deutlich verbesserte Reinigungswirkung bedingt.

Anstelle von Metallkationen können auch Ammoniumkationen verwendet werden. Hierbei besteht allerdings die Gefahr, dass bei dem elektrolytischen Prozess andere Ammoniumverbindungen (z. B. Ammoniak) gebildet werden. Dies stellt eine Gefährdung des Patienten dar und zeigt sich auch durch einen sehr unangenehmen Geschmack und Geruch.

Es wurde bei Versuchen beobachtet, dass sich der Biofilm teilweise in sehr kleinen Fragmenten oder aber in größeren zusammenhängenden Teilen ablöst. Letzteres wird bevorzugt, da damit vergleichsweise großflächig sehr günstige Reinigungsergebnisse erzielt werden können. Untersuchungen haben weiterhin gezeigt, dass der Abtransport des gelösten Biofilms und/oder seiner Fragmente durch eine Schaumbildung an der Implantatoberfläche begünstigt wird. Es hat sich herausgestellt, dass es günstig ist, dass nach dem Einsatz eines Elektrolyten aus den beschriebenen Metallsalzen, Säuren und Wasser, die insbesondere für die Abtötung und Ablösung zuständig sind, ein zweiter Elektrolyt zum Einsatz kommt, welcher zusätzlich eine Schaumbildung im Bereich der Kathode aufweist. Eine solche Schaumbildung kann erreicht werden, indem dem Elektrolyten bevorzugt zusätzlich ein Stoff zugegeben wird, der mindestens drei CH₂-Kettenglieder oder mindestens ein CH₂-Kettenglied und mindestens eine Kohlenstoffringverbindung aufweist. Hierbei können z. B. Öl und/oder Chlorhexidin zum Einsatz kommen. Darüber hinaus können auch ionische Flüssigkeiten verwendet werden, welche vorzugsweise ein I⁻-, Cl⁻- und/oder OH⁻-Ionen aufweisen. Da der organische Kationenanteil einer ionischen Flüssigkeit unter Umständen auf der Implantatoberfläche reduziert wird und dort verbleicht, ist es in einer besonders günstigen Ausführung möglich, Knochenwachstumsfaktoren an diesen Kationenanteil anzuhängen.

Werden Chloride und Jodide im richtigen Verhältnis gemischt, kann die störende Chlorgasbildung vermieden werden. An der Anode entsteht:

2J + 5Cl + 6H₂O → 10HCl + 2HIO₃

Dies bedeutet, dass an der Anode Salzsäure sowie Jodsäure gebildet werden. Diese haben sicherlich eine starke antimikrobielle Wirkung und werden auch beim Zusammentreffen mit dem sich kathodisch bildenden Hydroxid wieder neutralisiert.

Eine ganz besonders bevorzugte Zusammensetzung der Behandlungsflüssigkeit, die im Laborversuch besonders günstige Reinigungseigenschaft zeigte, umfasst eine wässrige Lösung von Natriumiodid (Nal) oder Kaliumiodid (KI) in einem Mischungsverhältnis von mindestens 5, bevorzugt mindestens 10, besonders bevorzugt mindestens 20 g des Salzes pro 30 ml Flüssigkeit (d. h. Wasser, H₂O, gegebenenfalls mit CO₂ angereichert), durch Zugabe von Milchsäure gepuffert auf einen pH-Wert von etwa 2,7 bis 2,9.

Bei der Prozessführung ist eine mittlere Stromdichte am Pfostenteil 2 bzw. am behandlungsbedürftigen Bauteil von mindestens 50 mA/cm², vorteilhafterweise von mindestens 100 mA/cm², besonders bevorzugt von mindestens 250 mA/cm², vorgesehen, wobei diese Stromdichte auf die äußere Oberfläche des Pfostenteils 2 (also ohne Berücksichtigung von oberflächenvergößernden Eigenschaften wie beispielsweise Rauigkeit oder Oberflächenstruktur) bezogen ist. Zur Ablösung des Biofilms hat sich eine durchschnittliche Stromdichte von 50 mA/cm² bis 300 mA/cm², vorteilhafterweise von 100 mA/cm² bis 200 mA/cm² als besonders günstig erwiesen. Zum Abtransport der Biofilmfragmente sollte die mittlere Stromdichte vorzugsweise auf den Bereich von 300 mA/cm² bis 5.000 mA/cm² oder besonders vorteilhaft von 1.500 mA/cm² bis 2.000 mA/cm² erhöht werden.

Das Behandlungssystem 1 und insbesondere dessen Steuereinheit 18 ist für eine koordinierte Verfahrensführung in dem Sinne ausgelegt, dass die Einspeisung der Behandlungsflüssigkeit einerseits und die Strombeaufschlagung andererseits abgestimmt aufeinander vorgenommen werden. Dazu kann beispielsweise vorgesehen sein, dass über die Steuereinheit 18 eine koordinierte Ansteuerung der dem Verbindungsschlauch 6 oder dem Vorratsbehälter 8 zugeordneten Fördereinheit für die Behandlungsflüssigkeit einerseits und der elektrischen Versorgungseinheit 16 andererseits erfolgt.

### Bezugszeichenliste

- 1: Behandlungssystem
- 2: Medienkanüle
- 4: Auslassöffnung
- 6: Verbindungsschlauch
- 8: Vorratsbehälter
- 10: Leiterelement
- 12: Ende
- 14: Kontaktspitze
- 16: Versorgungseinheit
- 18: Steuereinheit
- 19: Kabel
- 20: Keramikkörper
- 22: Pfostenteil
- 24: Dentalimplantat
- 26: Aufbauteil
- 28: Verbindungsschraube
- 30: Zahnersatzstück
- 32: Außengewinde
- 34: Raumbereich
- 36: Kieferknochen
- 40: Oberflächenbereich
- 42: Beschichtung
- 44: Implantatschulter

## Patentansprüche

1. Keramikkörper (20) zur Verwendung in einem Knochenimplantat, insbesondere in einem Dentalimplantat (24), der vollständig aus einer elektrisch leitfähigen oder elektrisch halbleitenden Keramik gefertigt oder der in dem für den Kontakt mit menschlichem Gewebe vorgesehenen Oberflächenbereich (40) mit einer elektrisch leitfähigen Beschichtung (42) aus einer dotierten Keramik, aus einem elektrisch leitfähigen Kunststoff und/oder aus einem elektrisch leitfähigen Kohlenstoff versehen ist und im für den Kontakt mit menschlichem Gewebe vorgesehenen Oberflächenbereich (40) einen spezifischen elektrischen Widerstand von höchstens 10⁻² Ω cm aufweist.

2. Keramikkörper (20) nach Anspruch 1, der im für den Kontakt mit menschlichem Gewebe vorgesehenen Oberflächenbereich (40) einen spezifischen elektrischen Widerstand von höchstens 10⁻⁴ Ω cm, vorzugsweise von höchstens 10⁻⁵ Ω cm aufweist.

3. Keramikkörper (20) nach Anspruch 1 oder 2, bei dem die Beschichtung (42) von Indiumoxid und/oder Indium-Zinn-Oxid, von Zinkoxid, von Siliziumnitrit, von MoSi₂, von Si₃N₄-MoSi₂-SiC, von ZrO₂/CaO, von Al₂O₃/TiN, von BaTiO₃, von einem Silizid, von einem Nitrid, von einer titanhaltigen Keramik und/oder von einem Titanat gebildet ist.

4. Knochenimplantat mit einem Grundkörper, der als Keramikkörper (20) nach einem der Ansprüche 1 bis 3 ausgeführt ist.

5. Dentalimplantat (24) mit einem in einen Kieferknochen (36) einbringbaren Pfostenteil (22) und mit einem diesem zugeordneten Aufbauteil (26), wobei das Pfostenteil (22) als Keramikkörper (20) nach einem der Ansprüche 1 bis 3 ausgeführt ist.

6. Dentalimplantat (24) nach Anspruch 5, dessen Pfostenteil (22) im für den Kontakt mit menschlichem Gewebe vorgesehenen Oberflächenbereich(40) mit der elektrisch leitfähigen Beschichtung versehen ist, die im Bereich der Implantatschulter (44) eine im Vergleich zur sonstigen Oberfläche vergrößerte Schichtdicke aufweist.

7. Dentalimplantat (24) nach Anspruch 5 oder 6, dessen Pfostenteil (22) als Keramikkörper (20) auf Basis von Yttrium-und/oder Aluminiumoxid-stabilisiertem Zirkonoxid ausgeführt ist.

8. Dentalimplantat (24) nach Anspruch 7, dessen für den Kontakt mit menschlichem Gewebe vorgesehener Oberflächenbereich (40) zumindest in einem Teilbereich mit einer nanoskopische Poren aufweisenden oder anderweitig nanoskopisch ausgeführten Struktur versehen ist, und der eine Verarmungszone auf Basis von Yttrium- und/oder Aluminiumoxid stabilisiertem Zirkonoxid mit im Vergleich zum Innenvolumen reduziertem Yttrium- bzw. Aluminiumoxid-Anteil aufweist.

## Claims

1. A ceramic body (20) for use in a bone implant, in particular in a dental implant (24), which is made completely from an electrically conductive or electrically semiconductive ceramic material or which, in the surface area (40) intended for contact with human tissue, is provided with an electrically conductive coating (42) made from a doped ceramic material, an electrically conductive synthetic material and/or from an electrically conductive carbon, and which, in the surface area (40) intended for contact with human tissue, has a specific electrical resistance of maximally 10⁻² Ω cm.

2. The ceramic body (20) of claim 1, which, in the surface area (40) intended for contact with human tissue, has a specific electrical resistance of maximally 10⁻⁴ Ω cm, preferably of maximally 10⁻⁵ Ω cm.

3. The ceramic body (20) of claim 1 or 2, wherein the coating (42) is formed by indium oxide and/or indium tin oxide, by zinc oxide, by silicon nitrite, by MoSi₂, by Si₃N₄-MoSi₂-SiC, by ZrO₂/CaO, by Al₂O₃/TiN, by BaTiO₃, by a silicide, by a nitride, by a titanous ceramic material and/or by a titanate.

4. A bone implant having a base body designed as a ceramic body (20) according to any of claims 1 to 3.

5. A dental implant (24) having a post part (22) to be introduced into a jaw bone (36) and a mounting part (26) associated therewith, the post part (22) being designed as a ceramic body (20) according to any of claims 1 to 3.

6. The dental implant (24) of claim 5, whose post part (22) is provided, in the surface area (40) intended for contact with human tissue, with the electrically conductive coating whose layer thickness is enlarged in the area of the implant shoulder (44) as compared to the rest of the surface.

7. The dental implant (24) of claim 5 or 6, whose post part (22) is designed as a ceramic body (20) based on yttrium and/or aluminium oxide-stabilized zirconium oxide.

8. The dental implant (24) of claim 7, whose surface area (40) intended for contact with human tissue is provided, at least in a partial area, with a structure having nanoscopic pores or designed otherwise in a nanoscopic manner, and comprises a depletion zone based on yttrium and/or aluminium oxide-stabilized zirconium oxide which has a reduced share of yttrium or aluminium oxide as compared with the inner volume.

## Revendications

1. Corps céramique (20) pour utilisation dans un implant osseux, en particulier dans un implant dentaire (24), qui est fabriqué complètement d'une céramique conductrice électriquement ou semi-conductrice électriquement ou qui est pourvu, dans la région de surface (40) prévue pour le contact avec le tissu humain, d'un revêtement (42) conducteur électriquement, fait d'une céramique dopée, d'une matière synthétique conductrice électriquement et/ou d'un carbone conducteur électriquement, et qui a dans la région de surface (40) prévue pour le contact avec le tissu humain une résistance électrique spécifique de 10⁻² Ω cm au maximum.

2. Corps céramique (20) selon la revendication 1, qui a dans la région de surface (40) prévue pour le contact avec le tissu humain une résistance électrique spécifique de 10⁻⁴ Ω cm au maximum, de préférence de 10⁻⁵ Ω cm au maximum.

3. Corps céramique (20) selon la revendication 1 ou 2, dans lequel le revêtement (42) est formé par oxyde d'indium et/ou oxyde d'indium et d'étain, par oxyde de zinc, par nitrite de silicium, par MoSi₂, par Si₃N₄-MoSi₂-SiC, par ZrO₂/CaO, par Al₂O₃/TiN, par BaTiO₃, par un silicide, par un nitride, par une céramique titaneuse et/ou par un titanate.

4. Implant osseux ayant un corps de base conçu comme un corps céramique (20) selon l'une quelconque des revendications 1 à 3.

5. Implant dentaire (24) ayant un élément pilier (22) qui doit être introduit dans un os maxillaire (36) et un élément de montage (26) y affecté, l'élément pilier (22) étant conçu comme un corps céramique (20) selon l'une quelconque des revendications 1 à 3.

6. Implant dentaire (24) selon la revendication 5, dont l'élément pilier (22) est pourvu, dans la région de surface (40) prévue pour le contact avec le tissu humain, d'un revêtement conducteur électriquement dont l'épaisseur de couche est élargie dans la région de l'épaulement (44) de l'implant par rapport au reste de la surface.

7. Implant dentaire (24) selon la revendication 5 ou 6, dont l'élément pilier (22) est conçu comme un corps céramique (20) à base d'oxyde de zirconium stabilisé par oxyde d'yttrium et/ou par oxyde d'aluminium.

8. Implant dentaire (24) selon la revendication 7, dont la région de surface (40) prévue pour le contact avec le tissu humain est pourvue, au moins dans une région partielle, d'une structure ayant des pores nanoscopiques ou conçue autrement de façon nanoscopique, et comprend une zone de déplétion à base d'oxyde de zirconium stabilisé par oxyde d'yttrium et/ou par oxyde d'aluminium qui a une part d'oxyde d'yttrium ou d'aluminium réduite par rapport au volume intérieur.
